# EUROPEAN PATENT SPECIFICATION

(11) **EP 1 432 442 B1**
(45) Date of publication and mention of the grant of the patent: **03.01.2007**
(21) Application number: 02777259.9
(22) Date of filing: 30.09.2002
(51) Int. Cl.: A61K 39/39, A61K 9/127, A61K 9/00

(54) **The use of liposomes containing saponins and sterols in the manufacture of intradermal vaccines**
Verwendung von Liposomen mit Saponinen und Sterolen in der Herstellung von intradermalen Impfstoffen
Utilisation de liposomes contenant des saponines et stérols pour la fabrication de vaccins intradermique

(30) Priority: 01.10.2001 GB 0123580
(43) Date of publication of application: 30.06.2004
(73) Proprietor: GlaxoSmithKline Biologicals s.a., 1330 Rixensart (BE)
(72) Inventor: GARCON, Nathalie c/o GlaxoSmithKline Beecham, B-1330 Rixensart (BE)
(74) Representative: Dalton, Marcus Jonathan William
(86) International application number: PCT/EP2002/010931
(87) International publication number: WO 2003/028760

(56) References cited:
- WO-A-00/62800
- WO-A-02/32454
- WO-A-96/33739
- WO-A-98/15287
- WO-A-99/66043

## Description

The present invention provides novel uses for adjuvants in the preparation of vaccines to be administered intradermally. The adjuvants, of the present invention comprise a saponin and a sterol, wherein the saponin and sterol are formulated in a liposome. The intradermal adjuvants are used in the manufacture of intradermal vaccines for humans, and in the intradermal treatment of humans.

Current practice in vaccination is heavily biased towards intramuscular administration of vaccines. The intramuscular route has been studied extensively for decades, has a long track record of success for a number of reasons including the fact that the muscle is efficient at stimulating immune responses; also that it is easy and convenient and to deliver vaccines to the muscle in a reproducible manner.

In contrast, vaccination by other routes have proven either to be difficult to administer in a reproducible manner, or have had varied success in the induction of immune responses which arc equivalent to those achieved by the intramuscular route. For these reasons, the vast majority of vaccinations, particularly for non-live vaccines, are administered intramuscularly.

Intramuscular vaccines, however, are associated with significant drawbacks which make it desirable to develop other routes of vaccination. For example, intramuscular vaccines require administration of the vaccine deep into the tissue through long hypodermic needles, which leads to patient "needle-fear" and associated reduced vaccination regime compliance. In addition, some vaccines administered intramuscularly initiate significant local and systemic reactogenicity, such as local muscle inflammation or necrosis and associated pain, or systemic effects like headaches, nausea, or "flu-like" syndromes.

There is a need, therefore, to develop alternatives to intramuscular vaccination protocols, which are at least as efficient as, and preferably better than, intramuscular vaccination.

Saponins are taught in: Lacaille-Dubois, M and Wagner H. (1996. A review of the biological and pharmacological activities of saponins. Phytomedicine vol 2 pp 363-386). Saponins are steroid or triterpene glycosides widely distributed in the plant and marine animal kingdoms. Saponins are noted for forming colloidal suspensions in water which foam on shaking, and for precipitating cholesterol. When saponins are near cell membranes they create pore-like structures in the membrane which cause the membrane to burst. Haemolysis of erythrocytes is an example of this phenomenon, which is a property of certain, but not all, saponins.

Saponins are known as adjuvants in vaccines for systemic administration. The adjuvant and haemolytic activity of individual saponins has been extensively studied in the art (Lacaille-Dubois and Wagner, *supra*). For example, Quil A (derived from the bark of the South American tree Quillaja Saponaria Molina), and fractions thereof, are described in US 5,057,540 and "Saponins as vaccine adjuvants", Kensil, C. R., *Crit Rev Ther Drug Carrier Syst,* 1996, 12 (1-2):1-55; and EP 0 362 279 B1. Particulate structures, termed Immune Stimulating Complexes (ISCOMS), comprising fractions of Quil A are haemolytic and have been used in the manufacture of vaccines (Morein, B., EP 0 109 942 B1; WO 96/11711; WO 96/33739). The haemolytic saponins QS21 and QS17 (HPLC purified fractions of Quil A) have been described as potent systemic adjuvants, and the method of their production is disclosed in US Patent No.5,057,540 and EP 0 362 279 B1. Other saponins which have been used in systemic vaccination studies include those derived from other plant species such as Gypsophila and Saponaria (Bomford *et al.,* Vaccine, 10(9):572-577, 1992).

QS21 administered into the skin of mice has been described in Kensil *et al.,* 1991, *J.Immunol.,* 146(2): 431-7. It is not certain, however, that in this study whether the QS21 was administered to the dermis because of the technical limitations of the mouse skin model.

The saponin adjuvants are also required to be delivered to the muscle at a relatively high dose. The saponin adjuvants described above are to varying extents painful when delivered intramuscularly. There is also a need to improve the quality and magnitude of the immune response generated by intramuscular vaccines comprising a saponin.

The intradermal adjuvants described herein comprise a saponin and a sterol, wherein the saponin and sterol are formulated in a liposome. Use of these adjuvant formulations in the manufacture of intradermal vaccines for humans is provided by the present invention. Such human intradermal vaccines, surprisingly, stimulate significant immune responses against co-administered antigen that are of a magnitude at least as high as those induced by intramuscular administration, however, the use liposomes of the present invention require significantly less antigen to do so, and/or significantly less saponin adjuvant with associated reduction in reactogenic responses.

Preferred sterols include β-sitosterol, stigmasterol, ergosterol, ergocalciferol and cholesterol. These sterols are well known in the art, for example cholesterol is disclosed in the Merck Index, 11th Edn., page 341, as a naturally occurring sterol found in animal fat. The most preferred sterol is cholesterol.

Preferably the liposome is a unilamellar liposome. The adjuvant formulation preferably further comprises a lipid capable of forming a bilayer membrane. Accordingly, the liposomes preferably contain a neutral or zwitterionic lipid (for example phosphatidylcholine) which is preferably non-crystalline at room temperature, for example eggyolk phosphatidylcholine, dioleoyl phosphatidylcholine or dilauryl phosphatidylcholine, and of these lipids dioleoyl phosphatidylcholine is most preferred. The vesicles may also contain a charged lipid which increases the stability of the liposome structure for liposomes composed of saturated lipids. In these cases the amount of charged lipid is preferably 1-20% w/w, most preferably 5-10%.

The ratio of sterol to phospholipid is 1-50% (mol/mol), most preferably 20-25%.

Typically, if both are present, the sterol (cholesterol) : phosphatidylcholine ratio is (1:4 w/w).

The liposomes used in the present invention may be unilamellar or multilamellar. Most preferably the vesicles are unilamellar liposomes. The diameter of the vesicles as measured by dynamic light scattering techniques (such as for example measurement using a Malvern Zetasizer^{™} or Coulter Counter^{™}) is typically in the range of 10-1000 nm and more preferably between 10-220 nm, and more preferably between 10-150 nm in size and most preferably between 70-150 nm in diameter, such as around 115 nm (all ranges being expressed as size "by intensity"). Preferably at least 90% of the particles are within the specified size range, and most preferably at least 95% of the particles present are within the specified ranges.

Preferred saponins are those known in the art to be immunostimulatory. The saponins may be purified from natural sources, or be derivatives of saponins derived from natural sources. Alternatively the saponins may be hemi- or totally synthetic. Hemi-synthetic saponins may be assembled from other non-saponin chemicals.

The most preferred saponins are immunostimulatory purified, synthetic or hemi-synthetic saponins which may be derived from the bark of Quillaja Saponaria Molina. The compositions to be used contain an immunologically active saponin fraction from the bark of Quillaja Saponaria molina in substantially purified form. "Purified saponin" is intended to mean a substantially pure saponin which is purified to one or more of the following standards: 1) appearing as only one major carbohydrate staining band on silica gel TLC (EM Science HPTLC Si60) in a solvent system of 40mM acetic acid in chloroform/methanol/water (60/45/10 v/v/v); 2) appearing as only one major carbohydrate staining band on reverse phase TLC (EM Science Silica Gel RP-8) in a solvent system of methanol/water (70/30 v/v); or 3) appearing as only one major peak upon reverse phase HPLC on a vydac C4 (5micrometer particle size, 300 angstrom pore size, 4.6 mm ID X 25cm L) in 40mM acetic acid in methanol/water (58/42 v/v), or 3) at least 90% pure, as defined by being free from other components with which the saponin is normally associated in nature. Purity of saponin fractions can be measured using HPLC techniques described in US Patent No.5,057,540.
Preferably the compositions to be used in the invention contain the saponin fraction QS21. The QS21 is preferably in a substantially purified form, that is to say, as isolated by collection of a single HPLC peak after the separation of a saponin from the bark of Quillaja saponaria molina, or more specifically the QS21 is at least 90% pure, preferably at least 95% pure and most preferably at least 98% pure.

Other immunologically active saponin fractions useful in compositions of the invention include QA17/QS17. β-Escin is another preferred haemolytic saponin for use in the adjuvant compositions of the present invention. Escin is described in the Merck index (12^{th} ed: entry 3737) as a mixture of saponins occurring in the seed of the horse chestnut tree, Lat: *Aesculus hippocastanum.* Its isolation is described by chromatography and purification (Fiedler, *Arzneimittel-Forsch.* 4, 213 (1953)), and by ion-exchange resins (Erbring *et al.,* US 3,238,190). Fractions of escin, α and β, have been purified and shown to be biologically active (Yoshikawa M, et al. (*Chem Pharm Bull* (Tokyo) 1996 Aug;44(8):1454-1464)). β-escin is also known as aescin.

Another preferred saponin for use in the present invention is Digitonin. Digitonin is described in the Merck index (12^{th} Edition, entry 3204) as a saponin, being derived from the seeds of *Digitalis purpurea* and purified according to the procedure described Gisvold *et al., J.Am.Pharm.Assoc.,* 1934, 23, 664; and Ruhenstroth-Bauer, *Physiol.Chem.,* 1955, 301, 621. Its use is described as being a clinical reagent for cholesterol determination.

Small unilamellar vesicles (SUV) with a mean diameter particle size of between 70-150 nm comprising the saponin and the sterol (preferably QS21 and cholesterol) where there is excess sterol present are particularly preferred adjuvants for use in the present invention.

The ratio of saponin:sterol in the liposomal adjuvant formulations for use in the present invention will typically be in the order of 1 : 100 to 1 : 1 weight to weight. More preferably, excess sterol is present, and more preferably the ratio of saponin : sterol is at least 1 : 2 w/w, and most preferably the ratio will be 1:5 (w/w). In a preferred embodiment, when the saponin is QS21, the ratio of QS21 contained within the saponin fraction : sterol will typically be in the order of 1 : 100 to 1 : 1 weight to weight. Preferably excess sterol to QS21 is present, and more preferably the ratio of QS21 : sterol being at least 1 : 2 w/w, and most preferably the ratio will be 1:5 (w/w). In all of these disclosed ratios, cholesterol is the preferred sterol., and the ratios apply equally thereto.

Typically for human administration saponin and sterol will be present in a vaccine in the range of about 1 µg to about 100 µg, preferably about 10 µg to about 50 µg per dose.

Optionally, the adjuvants, and uses comprising them, further include an LPS derivative. Enterobacterial lipopolysaccharide (LPS) is a potent stimulator of the immune system, although its use in vaccines has been curtailed by its toxic effects. A non-toxic derivative of LPS, monophosphoryl lipid A (MPL), produced by removal of the core carbohydrate group and the phosphate from the reducing-end glucosamine, has been described by Ribi et al (1986, Immunology and Immunopharmacology of bacterial endotoxins, Plenum Publ. Corp., NY, p407-419) and has the following structure: 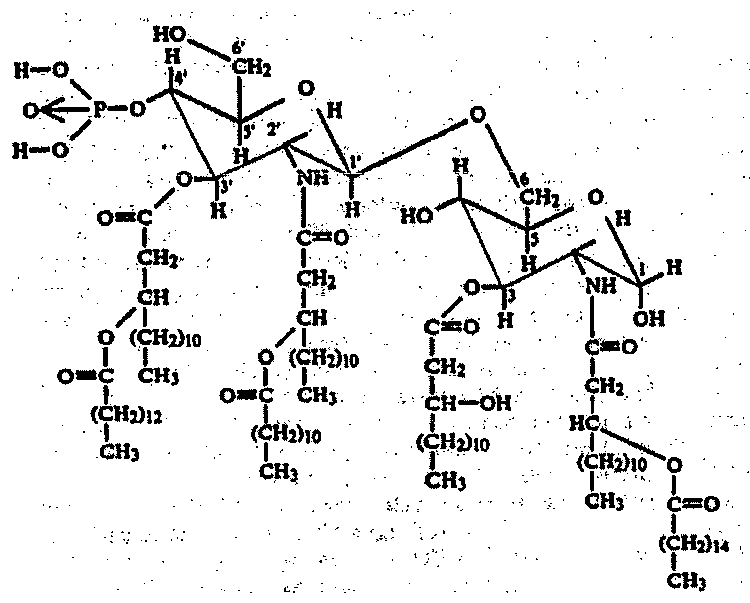 A further detoxified version ofMPL results from the removal of the acyl chain from the 3-position of the disaccharide backbone, and is called 3-O-Deacylated monophosphoryl lipid A (3D-MPL). It can be purified and prepared by the methods taught in GB 2122204B, which reference also discloses the preparation of diphosphoryl lipid A, and 3-O-deacylated variants thereof. A preferred form of 3D-MPL is in the form of an emulsion having a small particle size less than 0.2µm in diameter, and its method of manufacture is disclosed in WO 94/21292. Aqueous formulations comprising monophosphoryl lipid A and a surfactant have been described in WO 98/43670A2.

The bacterial lipopolysaccharide derived adjuvants which may be formulated in the adjuvants of the present invention may be purified and processed from bacterial sources, or alternatively they may be synthetic. For example, purified monophosphoryl lipid A is described in Ribi et al 1986 (supra), and 3-O-Deacylated monophosphoryl or diphosphoryl lipid A derived from *Salmonella sp.* is described in GB 2220211 and US 4912094. Other purified and synthetic lipopolysaccharides have been described (US 6,005,099 and EP 0 729 473 B1; Hilgers *et al.,* 1986, *Int.Arch.Allergy.Immunol.,* 79(4):392-6; Hilgers *et al.,* 1987, Immunology, 60(1):141-6; and EP 0 549 074 B1). Particularly preferred bacterial lipopolysaccharide adjuvants are 3D-MPL and the β(1-6) glucosamine disaccharides described in US 6,005,099 and EP 0 729 473 B1.

Accordingly, the LPS derivatives that may be used in the present invention are those immunostimulants that are similar in structure to that of LPS or MPL or 3D-MPL. In another aspect of the present invention the LPS derivatives may be an acylated monosaccharide, which is a sub-portion to the above structure of MPL.

A preferred disaccharide LPS derivative adjuvant, is a purified or synthetic lipid A of the following formula: 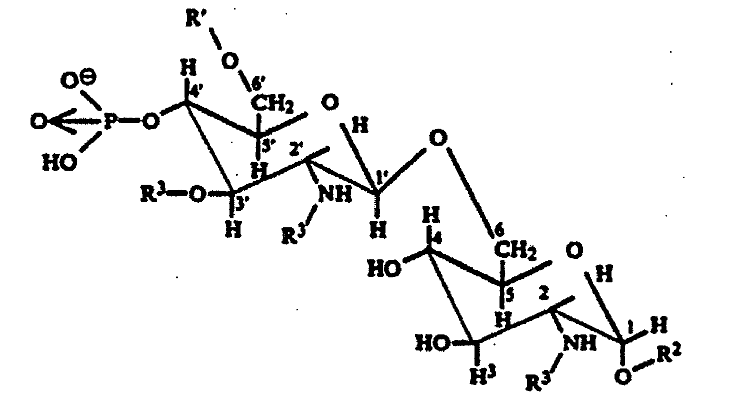 wherein R2 may be H or PO3H2; R3 may be an acyl chain or β-hydroxymyristoyl or a 3-acyloxyacyl residue having the formula: 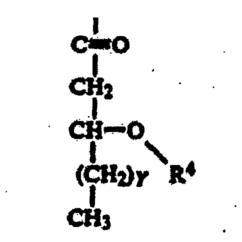 wherein 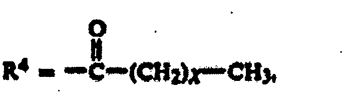 and wherein X and Y have a value of from 0 up to about 20.

The LPS derivative may be formulated with the saponin and sterol containing liposomes, or may be simply admixed with the saponin and sterol containing liposomes. Compositions of the invention, and uses thereof, are those wherein the sterol/saponin containing liposomes are initially prepared without the LPS derivative, and the LPS derivative is then added, preferably as particles with an average diameter of about 100 nm. In these embodiments the LPS derivative is therefore not contained within the vesicle membrane (known as LPS derivative-out). Compositions where an LPS derivative is contained within the liposome membrane (known as LPS derivative-in) also form an aspect of the invention. In this regard the adjuvant formulations preferably comprise a sterol and saponin containing liposome, and the LPS derivative (preferably 3D-MPL) is contained within the liposome membrane. Intradermal vaccine formulations comprising sterol, saponin and 3D-MPL in the membrane of a liposomal formulation are particularly potent in the induction of cell mediated immune responses, and form an alternative aspect of the present invention.

The antigen can be contained within the vesicle membrane or contained outside the vesicle membrane. Preferably hydrophilic antigens are outside and hydrophobic or lipidated antigens are either contained inside or outside the membrane structure. Alternatively, hydrophilic antigens may be outside the membrane structure but entrapped within the lumen of the vesicle.

More preferably, these adjuvant formulations comprise QS21 as the saponin, and 3D-MPL as the LPS derivative, and cholesterol as the sterol wherein the ratio of QS21:cholesterol is from 1:1 to 1:100 weight/weight, and most preferably 1:5 weight/weight. Such adjuvant formulations are described in EP 0 822 831 B, the disclosure of which is incorporated herein by reference.

In an alternative embodiment of the present invention there is provided the use of a sterol, a saponin (as described above) and an immunostimulatory oligonucleotide containing unmethylated CpG dinucleotides (CpG) in the manufacture of an intradermal vaccine for the treatment of a disease. Immunostimulatory oligonucleotides containing unmethylated CpG dinucleotides ("CpG") are known in the art as being adjuvants when administered by both systemic and mucosal routes (WO 96/02555, EP 468520, Davis *et al., J.Immunol,* 1998, 160(2):870-876; McCluskie and Davis, *J. Immunol.,* 1998, 161(9):4463-6). CpG is an abbreviation for cytosine-guanosine dinucleotide motifs present in DNA. The central role of the CG motif in immunostimulation was later elucidated in a publication by Krieg, Nature 374, p546 1995.

The preferred oligonucleotides for use in adjuvants or vaccines of the present invention preferably contain two or more dinucleotide CpG motifs separated by at least three, more preferably at least six or more nucleotides. The oligonucleotides of the present invention are typically deoxynucleotides. In a preferred embodiment the internucleotide in the oligonucleotide is phosphorodithioate, or more preferably a phosphorothioate bond, although phosphodiester and other internucleotide bonds are within the scope of the invention including oligonucleotides with mixed internucleotide linkages. Methods for producing phosphorothioate oligonucleotides or phosphorodithioate are described in US5,666,153, US5,278,302 and WO95/26204.

Examples of preferred oligonucleotides have the following sequences. The sequences preferably contain phosphorothioate modified internucleotide linkages. OLIGO 1(SEQ ID NO:1): TCC ATG ACG TTC CTG ACG TT (CpG 1826) OLIGO 2 (SEQ ID NO:2): TCT CCC AGC GTG CGC CAT (CpG 1758) OLIGO 3(SEQ ID NO:3): ACC GAT GAC GTC GCC GGT GAC GGC ACC ACG OLIGO 4 (SEQ ID NO:4): TCG TCG TTT TGT CGT TTT GTC GTT (CpG 2006) OLIGO 5 (SEQ ID NO:5): TCC ATG ACG TTC CTG ATG CT (CpG 1668)
Alternative CpG oligonucleotides may comprise the preferred sequences above in that they have inconsequential deletions or additions thereto. The CpG oligonucleotides utilised in the present invention may be synthesized by any method known in the art (eg EP 468520). Conveniently, such oligonucleotides may be synthesized utilising an automated synthesizer.

The oligonucleotides utilised in the present invention are typically deoxynucleotides. In a preferred embodiment the internucleotide bond in the oligonucleotide is phosphorodithioate, or more preferably phosphorothioate bond, although phosphodiesters are within the scope of the present invention. Oligonucleotide comprising different internucleotide linkages are contemplated, e.g. mixed phosphorothioate phophodiesters. Other internucleotide bonds which stabilise the oligonucleotide may be used.

As used herein, the term "intradermal delivery" means delivery of the vaccine to the dermis in the skin. However, the vaccine will not necessarily be located exclusively in the dermis. The dermis is the layer in the skin located between about 1.0 and about 2.0 mm from the surface in human skin, but there is a certain amount of variation between individuals and in different parts of the body. In general, it can be expected to reach the dermis by going 1.5 mm below the surface of the skin. The dermis is located between the stratum corneum and the epidermis at the surface and the subcutaneous layer below. Depending on the mode of delivery, the vaccine may ultimately be located solely or primarily within the dermis, or it may ultimately be distributed within the epidermis and the dermis.

The conventional technique of intradermal injection, the "mantoux procedure", comprises steps of cleaning the skin, and then stretching with one hand, and with the bevel of a narrow gauge needle (26-31 gauge) facing upwards the needle is inserted at an angle of between 10-15°. Once the bevel of the needle is inserted, the barrel of the needle is lowered and further advanced whilst providing a slight pressure to elevate it under the skin. The liquid is then injected very slowly thereby forming a bleb or bump on the skin surface, followed by slow withdrawal of the needle.

More recently, devices that are specifically designed to administer liquid agents into or across the skin have been described, for example the devices described in WO 99/34850 and EP 1092444, also the jet injection devices described for example in WO 01/13977; US 5,480,381, US 5,599,302, US 5,334,144, US 5,993,412, US 5,649,912, US 5,569,189, US 5,704,911, US 5,383,851, US 5,893,397, US 5,466,220, US 5,339,163, US 5,312,335, US 5,503,627, US 5,064,413, US 5,520, 639, US 4,596,556, US 4,790,824, US 4,941,880, US 4,940,460, WO 97/37705 and WO 97/13537. Alternative methods of intradermal administration of the vaccine preparations may include conventional syringes and needles, or devices designed for ballistic delivery of solid vaccines (WO 99/27961), or transdermal patches (WO 97/48440; WO 98/28037); or applied to the surface of the skin (transdermal or transcutaneous delivery WO 98/20734 ; WO 98/28037).

In the present invention, liposomes are used in the manufacture of a medicament for the treatment of individuals suffering from a disease or chronic disorder comprising the administration into the dermis of the individual a composition comprising a liposomal composition wherein the liposome comprises a sterol and an immunologically active saponin as described herein. Preferably the saponin is a purified or synthetic saponin from Quillaja Saponaria bark, and cholesterol adjuvants are formulated in a unilamellar liposome. The preferred methods of treatment are treatments of diseases caused by the following pathogens: human papilloma virus; Respiratory Syncytial Virus; hepatitis B and/or hepatitis A virus(es); meningitis B; meningococci and/or Haemophilus influenzae b and/or other antigens; Streptococcus pneumoniae; Varicella Zoster Virus.

Preferably the method of treatment also comprises the addition of an LPS derivative or CpG to the adjuvant formulation.

The vaccine formulations used in the present invention contain an antigen or antigenic composition capable of eliciting an immune response against a human pathogen, which antigen or antigenic composition is derived from HIV-1, (such as tat, nef, gp120 or gp160), human herpes viruses (HSV), such as gD or derivatives thereof or Immediate Early protein such as ICP27 from HSV1 or HSV2, cytomegalovirus (CMV (esp Human)(such as gB or derivatives thereof), Rotavirus (including live-attenuated viruses), Epstein Barr virus (such as gp350 or derivatives thereof), Varicella Zoster Virus (VZV, such as gpI, II and IE63), or from a hepatitis virus such as hepatitis B virus (for example Hepatitis B Surface antigen or a derivative thereof), hepatitis A virus (HAV), hepatitis C virus and hepatitis E virus, or from other viral pathogens, such as paramyxoviruses: Respiratory Syncytial virus (RSV, such as F and G proteins or derivatives thereof), parainfluenza virus, measles virus, mumps virus, human papilloma viruses (HPV, for example HPV6, 11, 16, 18), flaviviruses (e.g. Yellow Fever Virus, Dengue Virus, Tick-borne encephalitis virus, Japanese Encephalitis Virus) or Influenza virus (whole live or inactivated virus, split influenza virus, grown in eggs or MDCK cells, or whole flu virosomes (as described by R. Gluck, Vaccine, 1992, 10, 915-920) or purified or recombinant proteins thereof, such as HA, NP, NA, or M proteins, or combinations thereof), or derived from bacterial pathogens such as Neisseria spp, including N. gonorrhea and N. meningitidis (for example capsular polysaccharides and conjugates thereof, transferrin-binding proteins, lactoferrin binding proteins, PilC, adhesins); S. pyogenes (for example M proteins or fragments thereof, C5A protease, lipoteichoic acids), S. agalactiae, S. mutans; H. ducreyi; Moraxella spp, including M catarrhalis, also known as Branhamella catarrhalis (for example high and low molecular weight adhesins and invasins); Bordetella spp, including B. pertussis (for example pertactin, pertussis toxin or derivatives thereof, filamenteous hemagglutinin, adenylate cyclase, fimbriae), B. parapertussis and B. bronchiseptica; Mycobacterium spp., including M. tuberculosis (for example ESAT6, Antigen 85A, -B or -C), M. bovis, M. leprae, M. avium, M. paratuberculosis, M. smegmatis; Legionella spp, including L. pneumophila; Escherichia spp, including enterotoxic E. coli (for example colonization factors, heat-labile toxin or derivatives thereof, heat-stable toxin or derivatives thereof), enterohemorragic E. coli, enteropathogenic E. coli (for example shiga toxin-like toxin or derivatives thereof); Vibrio spp, including V. cholera (for example cholera toxin or derivatives thereof); Shigella spp, including S. sonnei, S. dysenteriae, S. flexnerii; Yersinia spp, including Y. enterocolitica (for example a Yop protein), Y. pestis, Y. pseudotuberculosis; Campylobacter spp, including C. jejuni (for example toxins, adhesins and invasins) and C. coli; Salmonella spp, including S. typhi, S. paratyphi, S. choleraesuis, S. enteritidis; Listeria spp., including L. monocytogenes; Helicobacter spp, including H. pylori (for example urease, catalase, vacuolating toxin); Pseudomonas spp, including P. aeruginosa; Staphylococcus spp., including S. aureus, S. epidermidis; Enterococcus spp., including E. faecalis, E. faecium; Clostridium spp., including C. tetani (for example tetanus toxin and derivative thereof), C. botulinum (for example botulinum toxin and derivative thereof), C. difficile (for example clostridium toxins A or B and derivatives thereof); Bacillus spp., including B. anthracis (for example botulinum toxin and derivatives thereof); Corynebacterium spp., including C. diphtheriae (for example diphtheria toxin and derivatives thereof); Borrelia spp., including B. burgdorferi (for example OspA, OspC, DbpA, DbpB), B. garinii (for example OspA, OspC, DbpA, DbpB), B. afzelii (for example OspA, OspC, DbpA, DbpB), B. andersonii (for example OspA, OspC, DbpA, DbpB), B. hermsii; Ehrlichia spp., including E. equi and the agent of the Human Granulocytic Ehrlichiosis; Rickettsia spp, including R. rickettsii; Chlamydia spp., including C. trachomatis (for example MOMP, heparin-binding proteins), C. pneumoniae (for example MOMP, heparin-binding proteins), C. psittaci; Leptospira spp., including L. interrogans; Treponema spp., including T. pallidum (for example the rare outer membrane proteins), T. denticola, T. hyodysenteriae; or derived from parasites such as Plasmodium spp., including P. falciparum; Toxoplasma spp., including T. gondii (for example SAG2, SAG3, Tg34); Entamoeba spp., including E. histolytica; Babesia spp., including B. microti; Trypanosoma spp., including T. cruzi; Giardia spp., including G. lamblia; Leshmania spp., including L. major; Pneumocystis spp., including P. carinii; Trichomonas spp., including T. vaginalis; Schisostoma spp., including S. mansoni, or derived from yeast such as Candida spp., including C. albicans; Cryptococcus spp., including C. neoformans.
Other preferred specific antigens for *M. tuberculosis* are for example Tb Ra12, Tb H9, Tb Ra35, Tb38-1, Erd 14, DPV, MTI, MSL, mTTC2 and hTCC1 (WO 99/51748). Proteins for *M. tuberculosis* also include fusion proteins and variants thereof where at least two, preferably three polypeptides of *M. tuberculosis* are fused into a larger protein. Preferred fusions include Ra12-TbH9-Ra35, Erd14-DPV-MTI, DPV-MTI-MSL, Erd14-DPV-MTI-MSL-mTCC2, Erd14-DPV-MTI-MSL, DPV-MTI-MSL-mTCC2, TbH9-DPV-MTI (WO 99/51748).
Most preferred antigens for Chlamydia include for example the High Molecular Weight Protein (HWMP) (WO 99/17741), ORF3 (EP 366 412), and putative membrane proteins (Pmps). Other Chlamydia antigens of the vaccine formulation can be selected from the group described in WO 99/28475.
Preferred bacterial vaccines comprise antigens derived from *Streptococcus spp,* including *S. pneumoniae* (for example capsular polysaccharides and conjugates thereof, PsaA, PspA, streptolysin, choline-binding proteins) and the protein antigen Pneumolysin (Biochem Biophys Acta, 1989, 67, 1007; Rubins et al., Microbial Pathogenesis, 25, 337-342), and mutant detoxified derivatives thereof (WO 90/06951; WO 99/03884). Particularly preferred pneumococcal vaccines are those described in WO 00/56539.

The Pht (Poly Histidine Triad) antigens are preferred Streptococcus antigens for the uses, pharmaceutical preparations and methods of the present invention. The Pht (Poly Histidine Triad) family comprises proteins PhtA, PhtB, PhtD, and PhtE. The family is characterised by a lipidation sequence, two domains separated by a proline-rich region and several histidine triads, possibly involved in metal or nucleoside binding or enzymatic activity, (3-5) coiled-coil regions, a conserved N-terminus and a heterogeneous C terminus. It is present in all strains of pneumococci tested. Homologous proteins have also been found in other Streptococci and Neisseria. Preferred members of the family comprise PhtA, PhtB and PhtD. More preferably, it comprises PhtA or PhtD.

The most preferred Pht antigen is PhtD.

It is understood, however, that the terms Pht A, B, D, and E refer to proteins having sequences disclosed in the citations below as well as naturally-occurring (and man-made) variants thereof that have a sequence homology that is at least 90% identical to the referenced proteins. Preferably it is at least 95% identical and most preferably it is 97% identical.

With regards to the PhtX proteins, PhtA is disclosed in WO 98/18930, and is also referred to Sp36. As noted above, it is a protein from the polyhistidine triad family and has the type II signal motif of LXXC.

PhtD is disclosed in WO 00/37105, and is also referred to Sp036D. As noted above, it also is a protein from the polyhistidine triad family and has the type II LXXC signal motif.

PhtB is disclosed in WO 00/37105, and is also referred to Sp036B. Another member of the PhtB family is the C3-Degrading Polypeptide, as disclosed in WO 00/17370. This protein also is from the polyhistidine triad family and has the type II LXXC signal motif. A preferred immunologically functional equivalent is the protein Sp42 disclosed in WO 98/18930. A PhtB truncate (approximately 79kD) is disclosed in WO99/15675 which is also considered a member of the PhtX family.

PhtE is disclosed in WO00/30299 and is referred to as BVH-3. Other preferred bacterial vaccines comprise antigens derived from *Haemophilus spp.,* including *H. influenzae type B* ("Hib", for example PRP and conjugates thereof), *non typeable H. influenzae,* for example OMP26, high molecular weight adhesins, P5, P6, protein D and lipoprotein D, and fimbrin and fimbrin derived peptides (US 5,843,464) or multiple copy varients or fusion proteins thereof.
Derivatives of Hepatitis B Surface antigen are well known in the art and include, inter alia, those PreS1, PreS2 S antigens set forth described in European Patent applications EP-A-414 374; EP-A-0304 578, and EP 198-474. In one preferred aspect the vaccine formulation of the invention comprises the HIV-1 antigen, gp120, especially when expressed in CHO cells. In a further embodiment, the vaccine formulation of the invention comprises gD2t as hereinabove defined.
In a preferred embodiment of the present invention vaccines containing the claimed adjuvant comprise antigen derived from the Human Papilloma Virus (HPV) considered to be responsible for genital warts (HPV 6 or HPV 11 and others), and the HPV viruses responsible for cervical cancer (HPV16, HPV18 and others).
Particularly preferred forms of genital wart prophylactic, or therapeutic, vaccine comprise L1 particles or capsomers, and fusion proteins comprising one or more antigens selected from the HPV 6 and HPV 11 proteins E6, E7, L1, and L2.
The most preferred forms of fusion protein are: L2E7 as disclosed in WO 96/26277, and proteinD(1/3)-E7 disclosed in GB 9717953.5 (PCT/EP98/05285).
A preferred HPV cervical infection or cancer, prophylaxis or therapeutic vaccine, composition may comprise HPV 16 or 18 antigens. For example, L1 or L2 antigen monomers, or L1 or L2 antigens presented together as a virus like particle (VLP) or the L1 alone protein presented alone in a VLP or caposmer structure. Such antigens, virus like particles and capsomer are per se known. See for example WO94/00152, WO94/20137, WO94/05792, and WO93/02184.
Additional early proteins may be included alone or as fusion proteins such as E7, E2 or preferably E5 for example; particularly preferred embodiments of this includes a VLP comprising L1E7 fusion proteins (WO 96/11272).
Particularly preferred HPV 16 antigens comprise the early proteins E6 or E7 in fusion with a protein D carrier to form Protein D - E6 or E7 fusions from HPV 16, or combinations thereof; or combinations of E6 or E7 with L2 (WO 96/26277).
Alternatively the HPV 16 or 18 early proteins E6 and E7, may be presented in a single molecule, preferably a Protein D- E6/E7 fusion. Such vaccine may optionally contain either or both E6 and E7 proteins from HPV 18, preferably in the form of a Protein D - E6 or Protein D - E7 fusion protein or Protein D E6/E7 fusion protein.
The vaccine of the present invention may additionally comprise antigens from other HPV strains, preferably from strains HPV 31 or 33.
Vaccines for use in the present invention further comprise antigens derived from parasites that cause Malaria. For example, preferred antigens from *Plasmodia falciparum* include RTS,S and TRAP. RTS is a hybrid protein comprising substantially all the C-terminal portion of the circumsporozoite (CS) protein of *P. falciparum* linked via four amino acids of the preS2 portion of Hepatitis B surface antigen to the surface (S) antigen of hepatitis B virus. It's full structure is disclosed in the International Patent Application No. PCT/EP92/02591, published under Number WO 93/10152 claiming priority from UK patent application No.9124390.7. When expressed in yeast RTS is produced as a lipoprotein particle, and when it is co-expressed with the S antigen from HBV it produces a mixed particle known as RTS,S. TRAP antigens are described in the International Patent Application No. PCT/GB89/00895, published under WO 90/01496. A preferred embodiment of the present invention is the intradermal administration of a Malaria vaccine wherein the antigenic preparation comprises a combination of the RTS,S and TRAP antigens. Other plasmodia antigens that are likely candidates to be components of a multistage Malaria vaccine are *P. faciparum* MSP1, AMA1, MSP3, EBA, GLURP, RAP1, RAP2, Sequestrin, PfEMP1, Pf332, LSA1, LSA3, STARP, SALSA, PfEXP1, Pfs25, Pfs28, PFS27/25, Pfs16, Pfs48/45, Pfs230 and their analogues in Plasmodium spp.
The formulations used may also contain an anti-tumour antigen and be useful for the immunotherapeutic treatment of cancers. The formulations may also contain an anti-tumour antigen and be useful for the immunotherapeutic treatment of cancers. For example, the adjuvant formulation finds utility with tumour rejection antigens such as those for prostrate, breast, colorectal, lung, pancreatic, renal or melanoma cancers. Exemplary antigens include MAGE 1 , 3 and MAGE 4 or other MAGE antigens such as disclosed in WO99/40188, PRAME, BAGE, Lage (also known as NY Eos 1) SAGE and HAGE (WO 99/53061) or GAGE (Robbins and Kawakami, 1996, Current Opinions in Immunology 8, pps 628-636; Van den Eynde et al., International Journal of Clinical & Laboratory Research (submitted 1997); Correale et al. (1997), Journal of the National Cancer Institute 89, p293. Indeed these antigens are expressed in a wide range of tumour types such as melanoma, lung carcinoma, sarcoma and bladder carcinoma. In a preferred embodiment prostate antigens are utilised, such as Prostate specific antigen (PSA), PAP, PSCA (PNAS 95(4) 1735 -1740 1998), PSMA or, in a preferred embodiment an antigen known as Prostase. Other tumour associated antigens useful in the context of the present invention include: Plu -1 J Biol. Chem 274 (22) 15633 -15645, 1999, HASH -1, HasH-2, Cripto (Salomon et al Bioessays 199,21 61 -70,US patent 5654140) Criptin US patent 5 981 215,., Additionally, antigens particularly relevant for vaccines in the therapy of cancer also comprise tyrosinase and survivin. Mucin dervied peptides such as Muc1 see for example US 5744,144 US 5827, 666 WO 8805054, US 4,963,484. Specifically contemplated are Muc 1 derived peptides that comprise at least one repeat unit of the the Muc 1 peptide, preferably at least two such repeats and which is recognised by the SM3 antibody (US 6 054 438). Other mucin derived peptides include peptide from Muc 5.
Vaccination according to the present invention is also useful in combination with breast cancer antigens such as her 2/ Neu, mammaglobin (US patent 5668267) or those disclosed in WO/00 52165, WO99/33869, WO99/19479, WO 98/45328. Her 2 neu antigens are disclosed inter alia, in US patent 5,801,005. Preferably the Her 2 neu comprises the entire extracellular domain ( comprising approximately amino acid 1 -645) or fragmants thereof and at least an immunogenic portion of or the entire intracellular domain approximately the C terminal 580 amino acids. In particular, the intracellular portion should comprise the phosphorylation domain or fragments thereof. Such constructs are disclosed in WO00/44899.
Intradermal vaccination according to the present invention may be used for the prophylaxis or therapy of allergy. Such vaccinations would comprise allergen specific (for example Der p1) and allergen non-specific antigens (for example peptides derived from human IgE, including but not restricted to the stanworth decapeptide (EP 0 477 231 B1)).
Intradermal vaccination according to the present invention may also be used for the prophylaxis or therapy of chronic disorders others than allergy, cancer or infectious diseases. Such chronic disorders are diseases such as atherosclerosis, and Alzheimer. Antigens relevant for the prophylaxis and the therapy of patients susceptible to or suffering from Alzheimer neurodegenerative disease are, in particular, the N terminal 39 -43 amino acid fragment (Aβ of the amyloid precursor protein and smaller fragments. This antigen is disclosed in the International Patent Application No. WO 99/27944 - (Athena Neurosciences).

Preferred antigens for use in the present invention are selecting from the group consisting of RSV, Streptococcus (and in particular using the vaccines described in WO 00/56359 the contents of which are incorporated herein by reference), HSV, HAV, HBV, VZV, HPV, and CMV. In addition, at least two of the vaccines in this preferred restricted list may be combined to form preferred vaccine combinations; for example the combination of a Streptococcus and RSV vaccine, and a combination of an HPV and HSV vaccine, and a combination of an HBV and HAV vaccine. Another specific vaccine combination that may be used in this second aspect of the present invention include the Infanrix^{™} range, made by GlaxoSmithKline Biologicals. Such vaccines are based on a "core" combination of Diptheria toxin, Tetanus toxin, and *B*. *pertussis* antigens. This vaccine comprises a pertussis component (either killed whole cell *B. pertussis* or accellular *pertussis* which typically consists of two antigens - PT and FHA, and often 69kDa, optionally with one or both agglutinogen 2 or agglutinogen 3). Such vaccines are often referred to as DTPw (whole cell) or DTPa (acellular).

Particular combination vaccines within the scope of the invention include:
Diptheria-Tetanus- Pertussis-Hepatitis B (DTP-HB)
Diptheria-Tetanus-Hepatitis B (DT-HB)
Hib-Hepatitis B
DTP-Hib-Hepatitis B
IPV (inactivated polio vaccine)- DTP-Hib-Hepatitis B [e.g. Infanrix-Hexa TM - SmithKline Beecham Biologicals s.a.]
Diptheria-Tetanus- Pertussis-Hepatitis B-IPV (DTP-HB-IPV) [e.g. Infanrix-Penta TM - SmithKline Beecham Biologicals s.a.].
The pertussis component is suitably a whole cell pertussis vaccine or an acellular pertussis vaccine containing partially or highly purified antigens. The above combinations may optionally include a component which is protective against Hepatitis A. Preferably the Hepatitis A component is formalin HM-175 inactivated. Advantageously, the HM-175 is purified by treating the cultured HM-175 with trypsin, separating the intact virus from small protease digested protein by permeation chromatography and inactivating with formalin. Advantageously the Hepatitis B containing combination vaccine is a paediatric vaccine.
The most preferred antigens are selected from the following pathogens: human papilloma virus; Respiratory Syncytial Virus; hepatitis B and/or hepatitis A virus(es); meningitis B; meningococcal antigens and/or Haemophilus influenzae b and/or other antigens; Streptococcus pneumoniae antigens alone or in combination with other antigens; Varicella Zoster Virus.
Preferably, the vaccine composition to be used does not comprise an influenza antigen.

The amount of antigen in each vaccine dose is selected as an amount which induces an immunoprotective response without significant, adverse side effects in typical vaccinees. Such amount will vary depending upon which specific immunogen is employed and how it is presented. Conventionally, each vaccine dose comprises 1-1000 µg of the or each antigen, preferably 1-500 µg, preferably 1-100µg, most preferably 1 to 50µg. Preferably, the total dose of antigen is 1-500 µg, preferably 1-100µg, most preferably 1 to 50µg. In a preferred aspect of the present invention the resultant vaccine are "low dose" in that they comprise 10 to 100µg of the or each antigen per dose, more preferably 1 to 20 µg per dose, and most preferably about 10µg per dose. More preferably the total dose of antigen is between 1 and 50 µg, more preferably 1 to 20 µg per dose, and most preferably about 10µg per dose. An optimal amount for a particular vaccine can be ascertained by standard studies involving observation of appropriate immune responses in subjects. Following an initial vaccination, subjects may receive one or several booster immunisation adequately spaced.

Preferably the vaccine is in a liquid volume smaller than for conventional intramuscular vaccines as described herein ("low volume"), particularly a volume of between about 0.05 ml and 0.2 ml. Preferably the volume of a dose of vaccine according to the invention is between 0.025 ml and 2.5 ml, more preferably approximately 0.1 ml or approximately 0.2 ml. A 50 µl dose volume might also be considered. A 0.1 ml dose is approximately one fifth to one tenth of the volume of a conventional intramuscular human vaccine dose (conventionally between 0.5 and 1 ml). The volume of liquid that can be administered intradermally depends in part upon the site of the injection. For example, for an injection in the deltoid region, 0.1 ml is the maximum preferred volume whereas in the lumbar region a large volume e.g. about 0.2 ml can be given.

The present invention provides a use of a sterol, a saponin formulated in a liposome in the manufacture of a low dose and low volume intradermal vaccine formulation.

### Example 1. Immunogenicity ofsplit vaccines in mice.

### Split RSV formulations

The following series of experiments exemplifies that split RSV induces a potent immune response when administered by the intradermal (ID) route.

### Methods

### Split RSV

A sample of RSV was split using 2% Sodium Deoxycholate. The virus to be split was incubated with the detergent overnight at room temperature with slow stirring.

After splitting, the solutions were dialyzed against formulation buffer (PO4 10 mM/ NaCl 150 mM pH7.5) for removal of excess detergent.

### Analysis: Ultracentrifugation

After half filling a centrifuge tube with the 30% sucrose solution (450 µl), the sample (450 µl) to be analyzed was laid gently and carefully onto this sucrose cushion then run for 1 hour at 50,000 rpm at +4°C in a Beckman TL100 rotor. After centrifugation, the tube was drained in 3 parts The upper phase (300 µl) is referred to as the 'supernatant'. The middle phase (300 µl) is the interface phase between the sample and the sucrose cushion, called herein the 'middle'. The lower phase (300µl) is the bottom solution with the resuspended pellet when centrifugation has been performed on integer virus; called the 'pellet'.
These 3 fractions were further analysed by Western blot against specific vial antigens. This analysis allows the integrity of the virus to be checked (pellet fraction positive) and the efficacy of the split to be determined (suitably, supernatant fraction positive for all or most structural proteins such as the envelope proteins).

### FG specific ELISA

The first immune read outs used to evaluate the immune response were ELISA assays which measure the total RSV FG-specific immunoglobulin (Ig) present in the sera of vaccinated animals. In these assays 96 well dishes are coated with recombinant RSV FG antigen and the animal sera are serially diluted and applied to the coated wells. Bound antibody is detected by addition of a Horseradish peroxidase bound anti-guinea pig Ig. Bound antibody is revealed upon addition of OPDA substrate, followed by treatment with 2 N H₂SO₄ and measurement of the optical density (OD)at 490 nm. The antibody titer is calculated from a reference using SoftMax Pro software (using a four parameter equation) and expressed in EU/ml.

### Neutralisation assay

In addition to ELISA assays, neutralization assays were included to further characterize the quality of the immune response induced by the immunizations. For the neutralization assay, two-fold dilutions of animal sera were incubated with RSV/A virus (3000 pfu) and guinea pig complement for 1 hour at 37°C in 96 well tissue culture dishes. Hep-2 cells (10⁴ cells/well) were added directly to each well and the plates incubated for 4 days at 37°C. The supernatants were aspirated and a commercially available WST-1 solution was added to each well. The plates were incubated for an additional 18-24 hours at 37°C. The OD was monitored at 450 nm and the titration analysed by linear regression analysis. The reported titer is the inverse of the serum dilution which resulted in 50% reduction of the maximal OD observed for uninfected cells.

### Preparation of vaccine

### 1.1 Method of preparation of liposomes:

A mixture of lipid (such as phosphatidylcholine) and cholesterol in organic solvent, is dried down under vacuum (or alternatively under a stream of inert gas). An aqueous solution (such as phosphate buffered saline) is then added, and the vessel agitated until all the lipid is in suspension. This suspension is then microfluidised until the liposome size is reduced to 100 nm, and then sterile filtered through a 0.2 µm filter.

Typically the cholesterol:phosphatidylcholine ratio is 1:4 (w/w), and the aqueous solution is added to give a final cholesterol concentration of 5 to 50 mg/ml.

The liposomes have a defined size of 100 nm and are referred to as SUV (for small unilamelar vesicles). If this solution is repeatedly frozen and thawed the vesicles fuse to form large multilamellar structures (MLV) of size ranging from 500nm to 15 µm. If 3D-MPL in organic solution is added to the lipid in organic solution the final liposomes contain 3D-MPL in the membrane (referred to as 3D-MPL in).

The liposomes by themselves are stable over time and have no fusogenic capacity.

### 1.2 Formulation procedure:

QS21 in aqueous solution is added to the liposomes. This mixture is then added to the antigen solution.

### Vaccination protocol

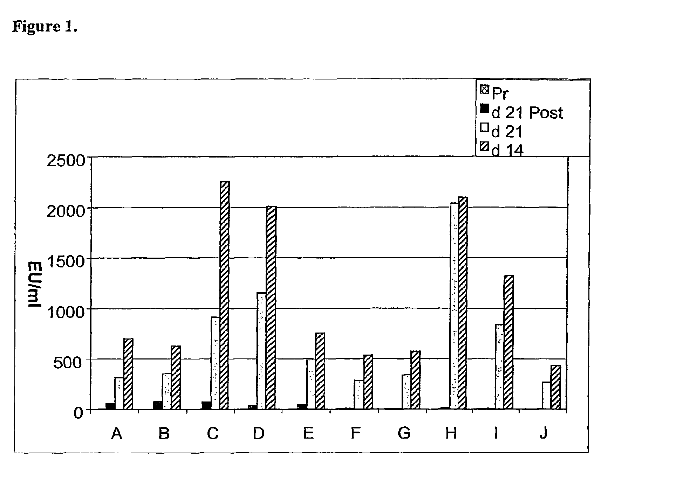

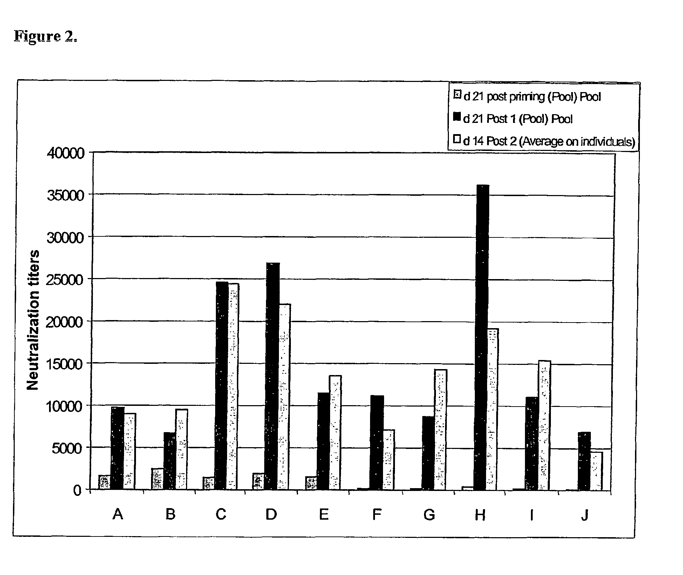

The immunogenicity of the RSV split antigen when administered by ID route was evaluated in guinea pigs. The feasibility of true ID injection in this species has been confirmed by injection of India ink into the dermis and histological examination of the tissues (data not shown) using the mantoux proceedure with a tuberculin needle. Again, in an effort to simulate the immune status found in elderly populations (i.e. primed against RSV), the Hartley guinea pigs (5 per group) were primed either with live RSV virus (5 X 10⁵ pfu administered IN in 100 µl - 50 µl/nostril; Groups A-E) or with purified whole RSV virus (containing 6 µg F protein administered IM in 100 µl; Groups F-J). Two equivalent doses of vaccine were administered at Day 21 and Day 42 post priming. Groups A and F received the split RSV preparation containing 4.2 µg of F protein administered by the ID route. Groups B and G received the split RSV preparation containing 0.84 µg of F protein administered by the ID route. Groups C and H received the split RSV preparation containing 4.2 µg of F protein adjuvanted with the saponin/sterol liposomes of the present invention (comprising 5µg QS21, 25µg cholesterol, phosphatidyl choline and 5µg 3D-MPL in the membrane of the liposome) administered in the ID route. Groups D and I received the split RSV preparation containing 0.84 µg of F protein adjuvanted with the same adjuvant as groups C administered by the ID route. Groups E and J received the split RSV preparation containing 4.2 µg of F protein administered by the IM route. Animals were bled 3 weeks after the first dose of vaccine and 2 weeks after the second dose of vaccine and the immune response evaluated.
The results of this experiment are summarised in Figures 1 and 2. Figure 1 shows the FG specific immune response detected in the guinea pig sera during the course of the experiment. Figure 2 summarises the neutralization data. Thus, in a primed population (primed either by live virus infection or administration of purified whole virus) a single dose of split RSV administered by the ID route is strongly immunogenic and this response can be further boosted by a second dose of vaccine.

## Claims

1. Use of a liposome comprising an immunologically active saponin, a sterol, and an antigen or antigenic preparation, in the manufacture of an intradermal vaccine to be administered intradermally for the treatment of disease.

2. Use as claimed in claim 1 wherein the immunologically active saponin is QS21

3. Use as claimed in claim 1 or 2 wherein the sterol is cholesterol

4. Use as claimed in claim 2 or 3, wherein the ratio of QS21:cholesterol is between 1:1 to 1:100 w/w.

5. Use as claimed in claim 1 wherein the liposome is a unilamellar liposome.

6. Use as claimed in claim 1 wherein the mean diameter of the liposomes is between 10-220 nm.

7. Use as claimed in any one of claims 1 to 6 wherein the intradermal vaccine further comprises a LPS derivative or an immunostimulatory CpG oligonucleotide.

8. Use as claimed in any one of claims 1 to 7, wherein the antigen or antigenic preparation is an antigen capable of generating an immune response against at least one of the group of pathogens consisting of Human Immunodeficiency Virus, Varicella Zoster virus, Herpes Simplex Virus type 1, Herpes Simplex virus type 2, Human cytomegalovirus, Dengue virus, Hepatitis A, B, C or E, Respiratory Syncytial virus, human papilloma virus, Influenza virus, Haemophilus Influenzae, Meningococcus, Salmonella, Neisseria, Borrelia, Chlamydia, Bordetella, Streptococcus, Mycoplasma, Mycobacteria, Plasmodium or Toxoplasma.

## Patentansprüche

1. Verwendung eines Liposoms, das ein immunologisch aktives Saponin, ein Sterol und ein Antigen oder eine antigene Zubereitung umfaßt, in der Herstellung eines intradermalen Impfstoffes zur intradermalen Verabreichung zur Behandlung einer Krankheit.

2. Verwendung nach Anspruch 1, worin das immunologisch aktive Saponin QS21 ist.

3. Verwendung nach Anspruch 1 oder 2, worin das Sterol Cholesterol ist.

4. Verwendung nach Anspruch 2 oder 3, worin das Verhältnis von QS21:Cholesterol zwischen 1:1 und 1:100 G/G ist.

5. Verwendung nach Anspruch 1, worin das Liposom ein einschichtiges Liposom ist.

6. Verwendung nach Anspruch 1, worin der mittlere Durchmesser der Liposomen zwischen 10 und 220 nm ist.

7. Verwendung nach einem der Ansprüche 1 bis 6, worin der intradermale Impfstoff ferner ein LPS-Derivat oder ein immunstimulierendes CpG-Oligonukleotid umfaßt.

8. Verwendung nach einem der Ansprüche 1 bis 7, worin das Antigen oder die antigene Zubereitung ein Antigen ist, das in der Lage ist, eine Immunantwort gegen wenigstens ein Pathogen aus der Gruppe von Pathogenen zu erzeugen, die aus einem Humanimmunschwächevirus, einem Varizella Zoster-Virus, einem Herpes simplex-Virus Typ 1, einem Herpes simplex-Virus Typ 2, einem humanen Cytomegalovirus, einem Denguevirus, Hepatitis A, B, C oder E, einem respiratorischen Syncytiumvirus, einem humanem Papillomavirus, einem Influenza (Grippe)-Virus, Haemophilus influenzae, Meningococcus, Salmonella, Neisseria, Borrelia, Chlamydia, Bordetella, Streptococcus, Mycoplasma, Mycobakterien, Plasmodium oder Toxoplasma besteht.

## Revendications

1. Utilisation d'un liposome comprenant une saponine active d'un point de vue immunologique, un stérol, et un antigène ou une préparation antigénique, dans la fabrication d'un vaccin intradermique à administrer par voie intradermique pour le traitement d'une maladie.

2. Utilisation selon la revendication 1, **caractérisée en ce que** la saponine active d'un point de vue immunologique est la QS21.

3. Utilisation selon la revendication 1 ou 2, **caractérisée en ce que** le stérol est le cholestérol.

4. Utilisation selon la revendication 2 ou 3, **caractérisée en ce que** le rapport QS21 : cholestérol est de 1 :1 à 1 : 100 p/p.

5. Utilisation selon la revendication 1, **caractérisée en ce que** le liposome est un liposome unilamellaire.

6. Utilisation selon la revendication 1, **caractérisée en ce que** le diamètre moyen des liposomes est de 10 à 220 nm.

7. Utilisation selon l'une quelconque des revendications 1 à 6, **caractérisée en ce que** le vaccin intradermique comprend en outre un dérivé de LPS ou un oligonucléotide CpG immunostimulant.

8. Utilisation selon l'une quelconque des revendications 1 à 7, **caractérisée en ce que** l'antigène ou la préparation antigénique est un antigène apte à produire une réponse immunitaire contre au moins l'un du groupe des pathogènes constitué par le virus de l'immunodéficience humaine, l'herpesvirus varicellae, le virus de l'herpes de type 1, le virus de l'herpes de type 2, le cytomégalovirus humain, le virus de la dengue, l'hépatite A, B, C ou E, le virus respiratoire syncytial, le virus du papillome humain, le virus de la grippe, un Haemophilus Inlfuenzae, un méningocoque, une salmonelle, une Neisseria, une Borellia, une Chlamydia, une Bordetella, un streptocoque, un mycoplasme, une mycobactérie, un Plasmodium ou un toxoplasme.
